(19) Europäisches Patentamt
European Patent Office
Office européen des brevets

(11) **EP 4 470 575 A1**

(12) # EUROPEAN PATENT APPLICATION

(43) Date of publication:
**04.12.2024 Bulletin 2024/49**

(21) Application number: **23176596.7**

(22) Date of filing: **01.06.2023**

(51) International Patent Classification (IPC):
**A61M 1/16** *(2006.01)* **A61M 1/34** *(2006.01)*

(52) Cooperative Patent Classification (CPC):
**A61M 1/3417; A61M 1/1676;** A61M 1/1647;
A61M 2202/0445; A61M 2205/3334

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Fresenius Medical Care Deutschland GmbH**
**61352 Bad Homburg (DE)**

(72) Inventors:
• **Klewinghaus, Jürgen**
**61352 Bad Homburg (DE)**
• **Lannoy, Jean-Michel**
**61352 Bad Homburg (DE)**

(74) Representative: **Herrmann, Uwe**
**Lorenz Seidler Gossel**
**Rechtsanwälte Patentanwälte**
**Partnerschaft mbB**
**Widenmayerstraße 23**
**80538 München (DE)**

(54) **BLOOD TREATMENT DEVICE**

(57) The present invention relates to a blood treatment device, comprising a first hemofilter configured to increase a hemoconcentration of blood; a second hemofilter arranged fluidically downstream of the first hemofilter and configured to remove toxins from the blood into an albumin containing dialysate; a flow regulator arranged in a fluid line conducting fluid out of the first hemofilter; a flow sensor configured to measure a flow in a fluid line conducting dialysate into the second hemofilter and a flow in a fluid line conducting dialysate out of the second hemofilter; and a control unit configured to control the flow regulator depending on the measurement data of the flow sensor to set an outflow of dialysate out of the first hemofilter.

**Fig. 1** 788-23He/CJC

EP 4 470 575 A1

**Description**

The present invention relates to a blood treatment device for removing protein-bound toxins from blood flowing in an extracorporeal circuit. Such a blood treatment device may be used for supporting liver function and / or in the treatment of sepsis.

Known devices for supporting liver function or for the treatment of sepsis often require a rather complicated structural set-up causing a need for specialized training for medical personnel operating these devices.

The present invention strives to overcome the disadvantages of the prior art. In particular, it is an object of the present invention to provide a simpler and more user-friendly blood treatment device for removing protein-bound toxins from blood flowing in an extracorporeal circuit. One advantage of the invention is, for example, that no additional pumps are necessary.

This object is achieved by the invention as defined by the independent claims. Advantageous embodiments of the invention are the subject matter of the dependent claims.

A first aspect of the present invention relates to a blood treatment device, comprising:

- a first hemofilter configured to increase a hemoconcentration of blood;
- a second hemofilter arranged fluidically downstream of the first hemofilter and configured to remove toxins from the blood into an albumin containing dialysate;
- a flow regulator arranged in a fluid line conducting fluid out of the first hemofilter;
- a flow sensor configured to measure a flow in a fluid line conducting dialysate into the second hemofilter and a flow in a fluid line conducting dialysate out of the second hemofilter; and
- a control unit configured to control the flow regulator depending on the measurement data of the flow sensor to set an outflow of fluid out of the first hemofilter.

The flow sensor can comprise or consist of a dialysate pump, e.g. the dialysate pump P4 shown in the figures. This has the advantage that no additional flow sensor has to be provided besides the pump used for conducting dialysate.

Preferably, the first and second hemofilters are arranged in a cascade so that blood leaving the first hemofilter flows into the second hemofilter.

Conventional hemofiltration systems have struggled to remove protein-bound toxins, such as inflammatory or apoptotic mediators transported by proteins such as albumin. Such protein-bound toxins do not pass a regular hemofilter, because the proteins are too large to cross the membrane of the hemofilter.

The present invention thus provides a first hemofilter configured to increase a hemoconcentration of blood and a second hemofilter arranged fluidically downstream of the first hemofilter and configured to remove toxins from the (concentrated) blood leaving the first hemofilter into an albumin containing dialysate.

The pore size of the first hemofilter is preferably set such that protein-bound toxins are retained in the blood while water and solute toxins are removed from the blood. Thus, hemoconcentration in the blood is increased, causing a drop in pH and reducing the binding affinity between the blood proteins (e.g. albumin) and toxins (e.g. inflammatory mediators).

In the second hemofilter, the concentrated blood is exposed to fresh dialysate containing albumin. Thus, the toxins comprised in the concentrated blood leaving the first hemofilter will pass the membrane of the second hemofilter driven by the concentration gradient to bind to the albumin in the dialysate and thereby be removed from the blood.

Optionally in order to facilitate this process, a fluid may be added to the blood between the first hemofilter and the second hemofilter to increase the gradient in albumin concentration driving toxins across the membrane of the second hemofilter into the albumin containing dialysate and/or a fluid may be added downstream the second hemofilter to replace the removed fluid.

Furthermore, regional anticoagulation using citrate reduces the risk of clotting in the hemofilters forced by the hemoconcentration. Regional anticoagulation may also be used to increase the clearance of protein-bound toxins from the blood, since the addition of citrate to the blood flow, before it enters the first hemofilter reduces the pH which results in a release of toxins bound e.g. to albumin. Therefore, these toxins become freely available in the blood flow and can be cleared by passages through the first and second hemofilters.

Thus, a blood treatment device according to the present invention can comprise an anticoagulant line configured to supplement blood with an anticoagulant fluid, preferably citrate, prior to flowing into the first hemofilter and can preferably further comprise a supplementation line configured to supplement blood flowing out from the second hemofilter with an electrolyte fluid, preferably a calcium solution, prior to return to the patient.

As citrate also causes the release of calcium ions e.g. from albumin, a blood treatment device according to the present invention can comprise a calcium sensor configured to measure the amount of calcium ions in the blood leaving the first hemofilter. The control unit of the blood treatment device can be configured to control a citrate pump adding citrate to the blood depending on the measurement data (values) of the calcium sensor.

According to the present invention, a flow regulator is arranged in a fluid line conducting fluid out of the first hemofilter and a flow sensor configured to measure a flow in a fluid line conducting dialysate into the second hemofilter (Qin, 2) and a flow in a fluid line conducting dialysate out of the second hemofilter (Qeff, 2) and the control unit of the blood treatment devices is programmed to control the flow regulator depending on the measurement data of the flow sensor (in particular, Qin,2 and Qeff,2) to set an outflow of fluid out of the first hemofilter.

In other words, the control unit of a blood treatment device according to the present invention can be configured to control the flow rate of fluid removal from the blood by the first hemofilter based on the balance of dialysate inflow and outflow of the second hemofilter.

For example, the outflow rate of fluid out of the first hemofilter can be set by the control unit to be equal to the flow rate of substitution fluid added plus a net ultrafiltration rate (UR rate).

The outflow rate of fluid out of the first hemofilter determines the hemoconcentration of the blood leaving the first hemofilter (higher outflow or removal rate, higher hemoconcentration) and thus determines the binding affinity between the protein-bound toxins and proteins present in the blood before the blood enters the second hemofilter.

In other words, setting the outflow rate of fluid out of the first hemofilter depending on the measured flow rates of albumin containing dialysate in and out of the second hemofilter allows for the hemoconcentration of the blood leaving the first hemofilter and entering the second hemofilter to be optimized for the removal of protein-bound toxins by the second hemofilter.

The control unit of a blood treatment device according to the present invention can be configured to compare a measurement value of the flow in the fluid line conducting dialysate into the second hemofilter (Qin, 2) to a measurement value of the flow in the fluid line conducting dialysate out of the second hemofilter (Qeff, 2) and to control the flow regulator depending on a detected positive or negative difference (e.g. +1 or -1) between the measurement values.

The flow sensor may be any sensor suitable for measuring flow, such as a noninvasive electromagnetic flow sensor. It may also be an ultrasonic Doppler type detector or a pressure sensor configured e.g. for detecting a differential pressure between two points in the dialysate flow, for derivation of a signal representative of the blood flow. In principle, additional sensors may be located at various points in the circuit.

The control unit can be configured to control the flow regulator depending on a detected difference between the measurement values by operating the flow regulator to increase flow by a defined increment if it is detected that the measurement value of the flow in the fluid line conducting dialysate into the second hemofilter (Qin, 2) is smaller than the measurement value of the flow in the fluid line conducting dialysate out of the second hemofilter (Qeff, 2).

Flow can be increased in a step-wise or continuous fashion. The defined increment can be stored in a memory unit of the blood treatment device and retrieved therefrom by the control unit based on the detected difference. Alternatively or additionally, the control unit can calculate the defined increment (e.g. open by +10 %) based on the detected difference.

Alternatively or additionally, the control unit can be configured to control the flow regulator depending on a detected difference between the measurement values by operating the flow regulator to decrease flow by a defined increment if it is detected that the measurement value of the flow in the fluid line conducting dialysate into the second hemofilter (Qin, 2) is larger than the measurement value of the flow in the fluid line conducting dialysate out of the second hemofilter (Qeff, 2).

Flow can be decreased in a step-wise or continuous fashion. The defined increment can be stored in a memory unit of the blood treatment device and retrieved therefrom by the control unit based on the detected difference. Alternatively or additionally, the control unit can calculate the defined increment (e.g. open by -10 %) based on the detected difference.

If no difference is detected (Qin, 2 = Qeff, 2), then the control unit can be configured not to change the setting of the flow regulator and maintain a current flow.

According to an embodiment of the invention, the first hemofilter is configured as a high filtration fraction ratio (high-FFR) hemofilter.

According to an embodiment of the invention, the second hemofilter is configured as a single-pass albumin dialysis (SPAD) hemofilter.

A blood treatment device according to the present invention can comprise a substitution line configured to supplement blood flowing out from the second hemofilter with a substitution fluid prior to return to the patient, for example to replenish a volume of fluid or electrolytes or other substances removed from the blood by the first hemofilter.

The flow regulator can comprise and / or consist of a clamp and / or occluding pump, in particular a roller pump. In other words, any structure suitable for regulating flow in a fluid line that can be controlled by a control unit is suitable as a flow regulator. The clamp can comprise a solenoid controlled by the control unit.

A flow regulator can also be arranged in the fluid line conducting dialysate out of the second hemofilter. For example, first and second clamps can be provided in the fluid line conducting fluid out of the first hemofilter and in the fluid line conducting spent albumin containing dialysate out of the second hemofilter.

The fluid line conducting fluid out of the first hemofilter and the fluid line conducting spent albumin containing dialysate out of the second hemofilter can both conduct fluid into a joint drainage line and the two clamps are preferably arranged between the first and second hemofilters and a branch point in that the effluent fluid lines meet the joint drainage line.

The control unit is preferably configured in this case to control the first and second clamps in a coordinated fashion based on the measurement data of the flow sensor measuring dialysate flow into and out of the second hemofilter.

A blood treatment device according to the present invention can comprise a conductivity sensor arranged in the fluid line conducting fluid out of the first hemofilter and configured to measure a conductivity of fluid flowing out of the first hemofilter. The conductivity of the fluid leaving the first hemofilter can give an indication of the hemoconcentration achieved by the first hemofilter.

The control unit can be configured to control an effluent pump and / or a substitution pump for providing substitution fluid based on a measurement value of the conductivity sensor.

The control unit can be configured to compare a measurement value of the conductivity sensor to a desired conductivity value and / or tolerance range and evaluate based on the comparison whether an extraction rate of the first hemofilter needs adjustment, e.g. whether the hemoconcentration resulting from extraction by the first hemofilter should be increased or decreased.

The control unit can be configured to control an effluent pump, e.g. the effluent pump P2 shown in the figures, based on a measurement value of the conductivity sensor, e.g. a flow rate of pump P2 may be increased.

The control unit can further be configured to control a substitution pump providing substitution fluid based on a measurement value of the conductivity and / or a measurement value of a flow rate of an effluent pump (e.g. the pump P2).

In other words, proper fluid balance can be maintained by controlling the substitution pump based on a measured flow rate of the effluent pump. As the effluent pump can be controlled based on the measured conductivity, the substitution pump can also be controlled based on the measured conductivity.

The substitution fluid (also called 'post-dilution volume') can be provided to add to the blood flow to compensate for volume and/or electrolytes or other substances lost as filtrate flow. The substitution fluid may be any sterile intravenous fluid having a desired concentration of electrolytes, such as a dialysate solution commonly known in the art. Additionally, the substitution fluid may be formulated as a buffer to maintain a desired acid-base balance. For example, the substitution fluid may be an acetate-based, lactate-based, citrate-based or bicarbonate-based buffer.

Another aspect of the invention pertains to a kit or retrofitting module for enabling a conventional blood treatment device to achieve the functionality of a blood treatment device according to the present invention. Such a module comprises a flow sensor as described above, a flow regulator as described above and a control unit as described above configured to control the flow regulator depending on the measurement data (in particular, measurement values) of the flow sensor to set an outflow of fluid out of a first hemofilter of the blood treatment device.

Furthermore, the present invention pertains to a method for controlling a flow regulator of a blood treatment device, wherein the flow regulator is arranged in a fluid line conducting fluid out of a first hemofilter of the blood treatment device, wherein the first hemofilter is configured to increase a hemoconcentration of blood and the blood treatment device further comprises a second hemofilter arranged fluidically downstream of the first hemofilter and configured to remove toxins from the blood into an albumin containing dialysate, comprising the steps: measuring a flow in a fluid line conducting dialysate into the second hemofilter and a flow in a fluid line conducting dialysate out of the second hemofilter by means of a flow sensor; and controlling the flow regulator by means of a control unit depending on the measurement data of the flow sensor to set an outflow (rate) of fluid out of the first hemofilter.

A method according to the present invention can further comprise the steps: comparing by means of a comparison unit a measurement value of the flow sensor representative of the flow in the fluid line conducting dialysate into the second hemofilter to a measurement value of the flow sensor representative of the flow in the fluid line conducting dialysate out of the second hemofilter and controlling by means of the control unit the flow regulator depending on a detected difference between the measurement values.

A method according to the present invention can further comprise the steps: operating the flow regulator by means of the control unit to increase flow by a defined increment if it is detected that the measurement value of the flow in the fluid line conducting dialysate into the second hemofilter ($Q_{in, 2}$) is smaller than the measurement value of the flow in the fluid line conducting dialysate out of the second hemofilter ($Q_{eff, 2}$)

Alternatively or additionally, a method according to the present invention can comprise the step: operating the flow regulator to decrease flow by a defined increment if it is detected that the measurement value of the flow in the fluid line conducting dialysate into the second hemofilter ($Q_{in, 2}$) is larger than the measurement value of the flow in the fluid line conducting dialysate out of the second hemofilter ($Q_{eff, 2}$).

The defined increments can be retrieved from a database or calculated as disclosed above. Generally, all features disclosed in the context of a device according to the present invention equally apply to a method of the present invention, although these features are not repeated explicitly to avoid redundancy.

It is to be noted that the present disclosure is not limited to the combinations of features explicitly disclosed therein, but comprises any other desirable combination of the disclosed features. In other words, features disclosed in the present application may be claimed in any desirable combination or in isolation.

Further features, advantages and technical effects of the present invention become apparent from the drawings showing preferred embodiments of the invention. The drawings merely serve purposes of illustrating the invention but do not purport to limit the invention. In the drawings, the same or similar components are denoted by the same reference signs.

In the drawings:

Fig. 1 illustrates the set-up of a blood treatment device according to a first embodiment.
Fig. 2 illustrates the set-up of a blood treatment device according to a second embodiment.
Fig. 3 illustrates the set-up of a blood treatment device according to a third embodiment.
Fig. 4 illustrates the set-up of a blood treatment device according to a fourth embodiment.

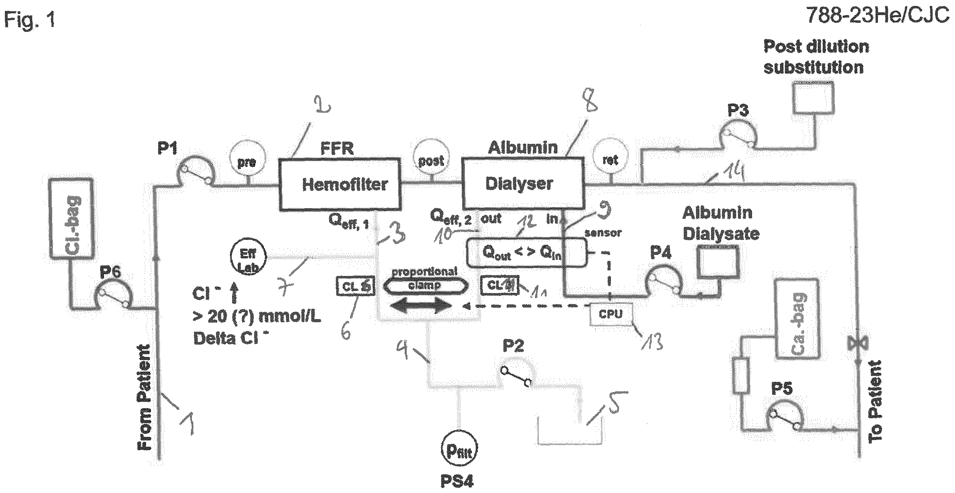

As shown in Fig. 1, a blood treatment device according to the present invention comprises a patient line 1 in that blood is pumped from a patient into the blood treatment device by the blood pump P1. Before the blood enters the first hemofilter 2, citrate is added to the blood by a citrate pump P6.

In the first hemofilter 2 fluid is removed from the blood and the hemoconcentration is raised. The fluid leaves the first hemofilter 2 via an effluent fluid line 3. The effluent fluid line 3 opens into a joint drainage line 4 so, that an effluent pump P2 pumps the fluid into a drain 5. The drained fluid can be measured e.g. by a scale. In the effluent fluid line 3 a clamp 6 is arranged to regulate the flow in the effluent fluid line 3.

Furthermore, a sampling line 7 branches off from the effluent fluid line 3. Samples taken at this point can be monitored for the content of chloride (Cl-) ions. A pressure sensor PS4 can be provided to measure filtration pressure in the drainage line 4 and in the second hemofilter 8.

After passing the first hemofilter 2, the blood flows into the second hemofilter or dialyser 8. A dialysate pump P4 is used to conduct fresh albumin containing dialysate through the dialyser 8 via an inlet line 9. Spent dialysate leaves the dialyser 8 via an outlet line 10 that opens into the joint drainage line 4. An optional clamp 11 is provided in the outlet line 10. The dialysate pump P4 may also provide a flow measurement signal.

A flow sensor 12 is provided to measure the fluid flow in the inlet line 9 and outlet line 10.

A control unit 13 retrieves measurement data such as measured value of flow rates from the flow sensor 12 and controls the clamps 6 and 11 acting as a proportional clamp accordingly in a coordinated manner. By this, a balance between fluid flow in the inlet line 9 and the outlet line 10 can be achieved.

The balance between fluid flow in the inlet line 9 and the outlet line 10 has to be readjusted when the filtration rate / hemoconcentration in the first hemofilter 2 is increased or decreased.

After passing the second dialyser 8, the blood flows in a return line 14 back to the patient. In the return line, substitution fluid is added to the blood via a substitution pump P3. Further downstream, calcium ions are replenished by an electrolyte pump P5.

In order to maintain the fluid balance of the treated patient, the flow $Q_{P2}$ of the effluent pump P2, which is extracting fluid from the patient, should be equal to the sum of the the flow $Q_{P4}$ of the dialysate pump P4 and the flow $Q_{eff,1}$ of the first hemofilter which is the sum of the flow $Q_{P3}$ of the substitution pump P3 and the flow $Q_{P5}$ of the electrolyte pump P5 and the flow $Q_{P6}$ of the citrate pump P6, which provides fluid to the patient. An intended net-Ultrafiltration flow must be added in this calculation as well.

$$Q_{P2} = Q_{P4} + Q_{eff,1} = Q_{P4} + (Q_{P3} + Q_{P5} + Q_{P6} + Q_{net\text{-}UF)}$$

The filtration fraction / hemoconcentration in the first hemofilter 2 can be adjusted by changing the flow of the effluent pump P2 and the substitution pump P3 accordingly, still maintaining the balance between the inflow and outflow of dialysate in and out of the second dialyser 8 by actuating clamps 6 and 11.

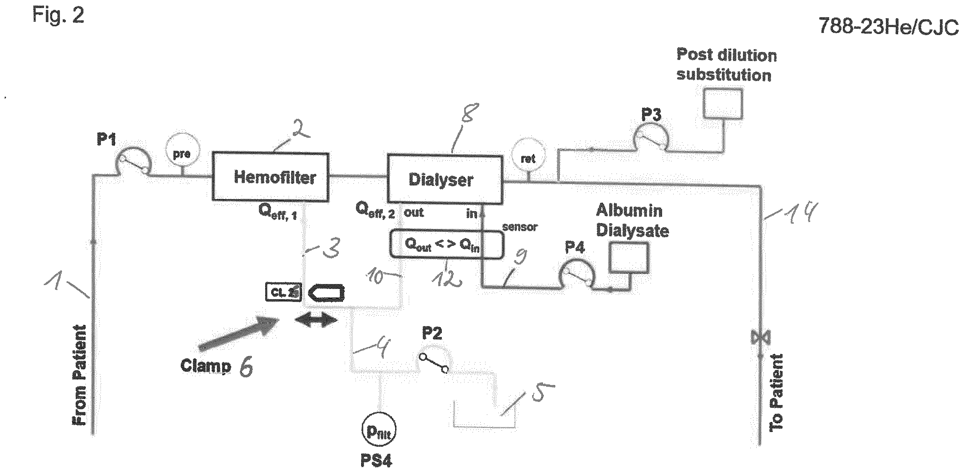

Fig. 2 shows an embodiment comprising a simpler flow regulator than the embodiment of Fig. 1. Instead of the proportional clamp comprising clamps 6 and 11 arranged in lines 3 and 10 of Fig. 1, in the embodiment of Fig. 2 the clamp 11 is omitted. Thus, the flow regulator is formed only by the clamp 6 in the effluent fluid line 3 in the embodiment of Fig. 2. By controlling clamp 6 based on the signal of sensor 12, it is possible to maintain the balance between the inflow and outflow of dialysate in and out of the second dialyser 8.

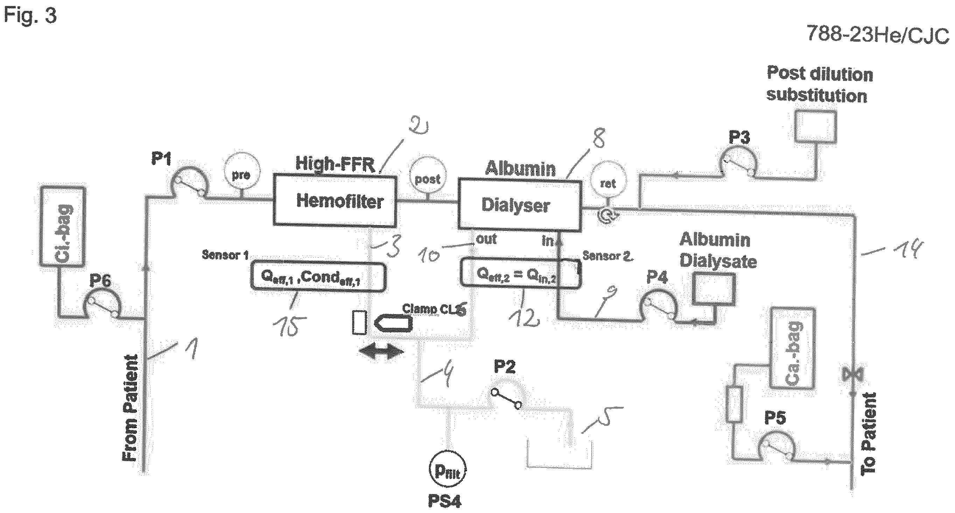

In the embodiment of Fig. 3, additionally a conductivity sensor 15 is present in the effluent fluid line 3. The conductivity sensor 15 can be configured to measure flow ($Q_{eff,1}$) and / or conductivity ($Cond\ eff,1$) of the fluid flowing in the effluent fluid line 3. This enables the user to check the effectiveness of the treatment and to adjust the substitution fluid (post-dilution). If the intended pH-shift and therefore shift in conductivity in $Q_{eff,1}$ is not observed, the filtration fraction ration could be increased by increasing the post-dilution flow flow of the substitution fluid and thereby the flow $Q_{eff,1}$.

Thus, in the embodiment of Fig. 3, two sensors can be present, i.e. the flow sensor 12 configured to measure flow in the outlet line 10 ($Q_{eff,1}$) and inlet line 9 ($Q_{in,2}$) of the dialyser 8 and the conductivity sensor 15 in the effluent fluid line 3.

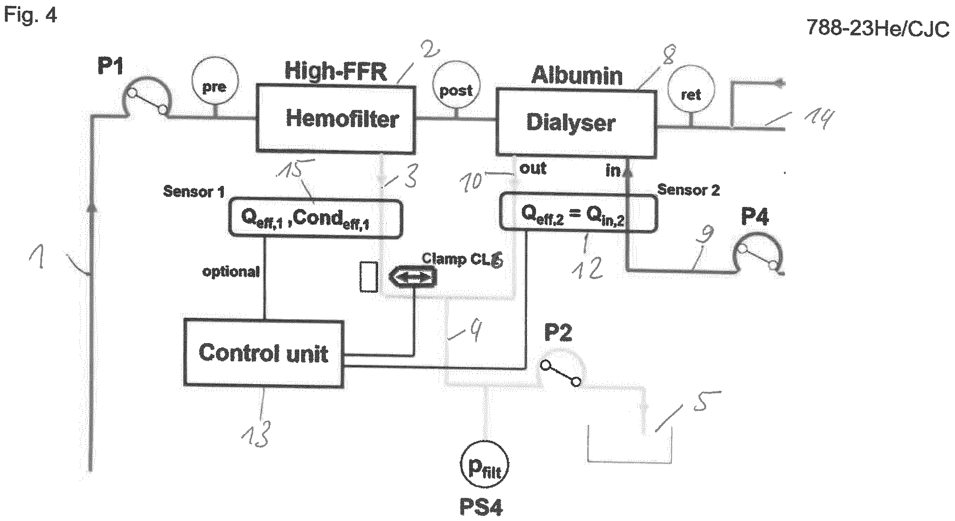

As shown in Fig. 4, the control unit can take into account measurement data or values of the conductivity sensor 15 and / or the flow sensor 12 to control the clamp 6 to regulate fluid flow in the fluid line 3. The measurement data of conductivity sensor 15 can indicate, if the targeted filtration fraction is achieved in hemofilter 2.

If the targeted conductivity is not achieved, the flow of effluent pump P2 may be adjusted, and by this the flow in effluent fluid line 3 and outlet line 10 will be affected. Still the balance between the inflow and outflow of dialysate in and out of the second dialyser 8 can be maintained by controlling clamp 6.

List of Reference Signs

1 patient line
2 first hemofilter
3 effluent fluid line
4 joint drainage line
5 drain
6 clamp
7 sampling line
8 second hemofilter or dialyser
9 inlet line
10 outlet line
11 clamp
12 flow sensor
13 control unit
14 return line
15 conductivity sensor
PS4 pressure sensor
P1 blood pump
P2 effluent pump
P3 substitution pump
P4 dialysate pump
P5 electrolyte pump
P6 citrate pump
$Q_{eff, 1}$ flow of effluent line 3 out of first hemofilter 2
$Q_{eff, 2}$ flow of the outlet line 10 out of second hemofilter 8
$Q_{in, 2}$ flow of the inlet line 9
$Cond_{eff, 1}$ conductivity of effluent line 3

**Claims**

1.  Blood treatment device, comprising:

    - a first hemofilter configured to increase a hemoconcentration of blood;
    - A second hemofilter arranged fluidically downstream of the first hemofilter and configured to remove toxins from the blood into an albumin containing dialysate;
    - a flow regulator arranged in a fluid line conducting fluid out of the first hemofilter;
    - a flow sensor configured to measure a flow in a fluid line conducting dialysate into the second hemofilter and a flow in a fluid line conducting dialysate out of the second hemofilter; and
    - a control unit configured to control the flow regulator depending on the measurement data of the flow sensor to set an outflow of fluid out of the first hemofilter.

2.  Blood treatment device according to claim 1, wherein the control unit is configured to compare a measurement value of the flow in the fluid line conducting dialysate into the second hemofilter to a measurement value of the flow in the fluid line conducting dialysate out of the second hemofilter and to control the flow regulator depending on a detected difference between the measurement values.

3.  Blood treatment device according to claim 2, wherein the control unit is configured to control the flow regulator depending on a detected difference between the measurement values by operating the flow regulator to increase flow by a defined increment if it is detected that the measurement value of the flow in the fluid line conducting dialysate into the second hemofilter (Qin, 2) is smaller than the measurement value of the flow in the fluid line conducting dialysate out of the second hemofilter (Qeff, 2).

4. Blood treatment device according to claim 2 or 3, wherein the control unit is configured to control the flow regulator depending on a detected difference between the measurement values by operating the flow regulator to decrease flow by a defined increment if it is detected that the measurement value of the flow in the fluid line conducting dialysate into the second hemofilter (Qin, 2) is larger than the measurement value of the flow in the fluid line conducting dialysate out of the second hemofilter (Qeff, 2).

5. Blood treatment device according to one of the preceding claims, wherein the first hemofilter is configured to be used as a high filtration fraction ratio (high-FFR) hemofilter.

6. Blood treatment device according to one of the preceding claims, wherein the second hemofilter is configured to be used as single-pass albumin dialysis (SPAD) hemofilter.

7. Blood treatment device according to one of the preceding claims, further comprising a substitution line configured to supplement blood flowing out from the second hemofilter with a substitution fluid prior to return to the patient.

8. Blood treatment device according to one of the preceding claims, wherein the flow regulator comprises a clamp and / or occluding pump, in particular a roller pump.

9. Blood treatment device according to one of the preceding claims, further comprising a flow regulator arranged in the fluid line conducting dialysate out of the second hemofilter.

10. Blood treatment device according to one of the preceding claims, further comprising a conductivity sensor arranged in the fluid line conducting fluid out of the first hemofilter and configured to measure a conductivity of fluid flowing out of the first hemofilter.

11. Blood treatment device according to claim 10, wherein the control unit is configured to compare a measurement value of the conductivity sensor to a desired conductivity value and / or tolerance range and evaluate based on the comparison whether an extraction rate of the first hemofilter needs adjustment.

12. Blood treatment device according to claim 10 or 11, wherein the control unit is configured to control an effluent pump based on a measurement value of the conductivity sensor.

13. Blood treatment device according to one of the preceding claims, wherein the control unit is further configured to control a substitution pump providing substitution fluid based on a measurement value of a flow rate of an effluent pump.

14. Blood treatment device according to one of the preceding claims, further comprising an anticoagulant line configured to supplement blood with an anticoagulant, preferably citrate, prior to flowing into the first hemofilter and preferably further comprising a supplementation line configured to supplement blood flowing out from the second hemofilter with an electrolyte fluid, preferably calcium solution, prior to return to the patient.

15. Blood treatment device according to one of the preceding claims, wherein the flow sensor comprises or consists of a dialysate pump.

16. Retrofitting module, for enabling a blood treatment device to achieve the functionality of a blood treatment device according to one of the preceding claims and / or to perform a method according to one of claims 17 to 19, comprising a flow sensor, a flow regulator and a control unit configured to control the flow regulator depending on the measurement data of the flow sensor to set an outflow of dialysate out of a first hemofilter of the blood treatment device.

17. Method for controlling a flow regulator of a blood treatment device, wherein the flow regulator is arranged in a fluid line conducting fluid out of a first hemofilter of the blood treatment device, wherein the first hemofilter is configured to increase a hemoconcentration of blood; and the blood treatment device further comprises a second hemofilter arranged fluidically downstream of the first hemofilter and configured to remove toxins from the blood into an albumin containing dialysate, comprising the steps:

measuring a flow in a fluid line conducting dialysate into the second hemofilter and a flow in a fluid line conducting dialysate out of the second hemofilter by means of a flow sensor; and

Controlling the flow regulator by means of a control unit depending on the measurement data of the flow sensor to set an outflow of fluid out of the first hemofilter.

18. Method according to claim 17, further comprising the steps: comparing by means of a comparison unit a measurement value of the flow in the fluid line conducting dialysate into the second hemofilter to a measurement value of the flow in the fluid line conducting dialysate out of the second hemofilter and controlling by means of the control unit the flow regulator depending on a detected difference between the measurement values.

19. Method according to claim 18, further comprising the steps: operating by means of the control unit the flow regulator to increase flow by a defined increment if it is detected that the measurement value of the flow in the fluid line conducting dialysate into the second hemofilter (Qin, 2) is smaller than the measurement value of the flow in the fluid line conducting dialysate out of the second hemofilter (Qeff, 2) and / or operating the flow regulator to decrease flow by a defined increment if it is detected that the measurement value of the flow in the fluid line conducting dialysate into the second hemofilter (Qin, 2) is larger than the measurement value of the flow in the fluid line conducting dialysate out of the second hemofilter (Qeff, 2).

Fig. 1

788-23He/CJC

EP 4 470 575 A1

Fig. 2

788-23He/CJC

Fig. 2

Post dilution substitution

P1  pre  **Hemofilter**  $Q_{eff,1}$  **Dialyser**  $Q_{eff,2}$  out  in  ret  P3

sensor

$Q_{out} <> Q_{in}$

Albumin Dialysate

P4

From Patient

Clamp  6

P2

$p_{filt}$  PS4

To Patient

CL

EP 4 470 575 A1

Fig. 3

788-23He/CJC

Post dilution substitution

P1 · pre · High-FFR · Hemofilter · post · Albumin · Dialyser · ret · P3

Cit.-bag

P6

From Patient

Sensor 1 · $Q_{eff,1}$, $Cond_{eff,1}$

15

Clamp CL6

out · in · Sensor 2 · $Q_{eff,2} = Q_{in,2}$

10 · 12

Albumin Dialysate

P4

9

14

Ca.-bag

P5

P2

$p_{filt}$

PS4

To Patient

EP 4 470 575 A1

11

Fig. 4

788-23He/CJC

EP 4 470 575 A1

Europäisches
Patentamt

European
Patent Office

Office européen
des brevets

# EUROPEAN SEARCH REPORT

Application Number

EP 23 17 6596

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/235682 A1 (UNIV WASHINGTON [US]) 10 November 2022 (2022-11-10) | 1-10, 13-19 | INV. A61M1/16 A61M1/34 |
| A | * page 9, line 7 - page 19, line 21; figures 1-10 * | 11,12 | |
| | ----- | | |
| A | EP 1 776 971 A1 (BELLCO SPA [IT]) 25 April 2007 (2007-04-25) * paragraphs [0013] - [0015]; figure 1 * | 1-19 | |
| | ----- | | |

TECHNICAL FIELDS
SEARCHED      (IPC)

A61M

The present search report has been drawn up for all claims

| Place of search | Date of completion of the search | Examiner |
|---|---|---|
| The Hague | 2 November 2023 | Schlaug, Martin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...............................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 17 6596

02-11-2023

| Patent document cited in search report | Publication date | Patent family member(s) | Publication date |
|---|---|---|---|
| WO 2022235682 A1 | 10-11-2022 | NONE | |
| EP 1776971 A1 | 25-04-2007 | NONE | |

EPO FORM P0459